# EUROPEAN PATENT SPECIFICATION

(11) **EP 3 658 470 B1**
(45) Date of publication and mention of the grant of the patent: **13.12.2023**
(21) Application number: 17918752.1
(22) Date of filing: 26.07.2017
(51) Int. Cl.: B65D 71/22, B65D 71/24, A61F 13/47, B65D 5/72, B65D 85/07, B65D 5/42

(54) **A DUAL-CHAMBER PACKAGE SYSTEM FOR ABSORBENT ARTICLES**
ZWEIKAMMERVERPACKUNGSSYSTEM FÜR ABSORBIERENDE ARTIKEL
SYSTÈME D'EMBALLAGE À DOUBLE CHAMBRE POUR ARTICLES ABSORBANTS

(43) Date of publication of application: 03.06.2020
(73) Proprietor: Tsinghua University, Beijing 100084 (CN); The Procter & Gamble Company, Cincinnati, OH 45202 (US)
(72) Inventor: ZHAO, Chao, Beijing 100084 (CN); LIU, Zhensheng, Beijing 100084 (CN); SUN, Xuejiao, Beijing 100084 (CN); WANG, Kaitian, Beijing 100084 (CN); JI, Lu, Beijing 100084 (CN); GAWARAN, Jayson Oxcena, Beijing 101312 (CN); DU, Minchen, Beijing 101312 (CN); MANDAL, Ankan, Mumbai Maharashtra 400099 (IN); CHEN, Jie, Beijing 101312 (CN); THORNTON, Mark Andrew, Beijing 101312 (CN)
(74) Representative: P&G Patent Germany
(86) International application number: PCT/CN2017/094429
(87) International publication number: WO 2019/019036

(56) References cited:
- EP-A1- 0 938 437
- CN-A- 101 854 900
- CN-A- 103 096 859
- CN-U- 202 807 340
- CN-U- 203 419 338
- CN-Y- 201 354 191
- US-A- 1 694 897
- US-A- 3 002 651
- US-A- 3 002 651
- US-A- 3 186 591
- US-A1- 2013 277 387
- US-A1- 2013 277 387

## Description

### FIELD OF THE INVENTION

The present invention relates to a dual-chamber package system for absorbent articles which is capable of self-standing.

### BACKGROUND OF THE INVENTION

Currently, absorbent articles such as diapers and feminine pads are sold in packages. Each absorbent article is folded along its lateral axis and optionally individually wrapped, and multiple folded/wrapped absorbent particles are then stacked together and compressed into a block, which is in turn packaged and sold in stores.

In one configuration, the package containing the absorbent articles can also function as a dispenser, i.e., it contains a single chamber for housing the block of absorbent articles and at least one opening which is formed by removing a detachable piece off said package and from which the absorbent particles can be pulled out one by one by the user. The opening is typically provided on the top surface or at one of the side walls of the package/dispenser. However, the problem with this design is that as the number of absorbent articles decreases due to usage, the top-most absorbent article inside the single chamber of the package/dispenser may fall below the opening. In this event, the user will need to stick his/her fingers into the opening to reach for the topmost absorbent article.

In order to make the absorbent articles more readily accessible to the user, another configuration provides a single-chambered package/dispenser with an opening at its bottom side. In this manner, the user will always pull out the bottom-most absorbent article and will not need to reach for such article, even if there is only one absorbent article left in the package/dispenser. In this configuration, there is still a need to provide a large enough clearance area in the bottom opening to allow sufficient contact between the user's hand and the bottom-most absorbent article, because the absorbent particles are compressed together inside the chamber, and it will be difficult to pull an individual article out without sufficient contact. The requirement for such sufficiently large clearance area at the bottom side of the package/dispenser renders such package/dispenser unsuitable for direct placement on a table or a counter. Instead, it will have to be mounted onto a wall or otherwise fixated to a surface via one of its side walls or even via its top surface, so as to leave the bottom side open and clear for dispensing the absorbent articles. Such a design is therefore much less versatile in its placement and requires more time and consideration for assembling and mounting.

There is therefore a need for an improved package/dispenser which enables easy access of the absorbent articles by the user, but which also is more versatile in placement and does not require mounting to a wall or fixation to a surface.

It is also advantageous to provide an improved package/dispenser with an opening characterized by an even larger clearance area, to allow better grip of the absorbent article by the user's hand or fingers.

US 3 002 651 A relates to dispensing packages for generally circular compressible soap or scouring pads.

US 3 186 591 A is concerned with dispenser boxes for articles, such as gum balls, candy, and pills including an integrally formed dispenser mechanism for expelling the articles one at a time.

US 1 694 897 A discloses distributing devices comprising a vertical channel to support and deliver cylindrical packages arranged in two vertical contacting columns.

US 2013/277387 A1 relates to cotton pad dispensers within which a number of disc-shaped cotton pads are stacked. The cotton pads are enclosed within a circular-cylindrical side wall. A first cut-out in a side wall extends upwards from the bottom end of the dispenser and a second cut-out provided in the bottom end wall extends from the first cut-out such that the cutouts comprise a mutual transition to form one combined withdrawal aperture.

### SUMMARY OF THE INVENTION

The inventors surprisingly and unexpectedly discover that introducing dual-chamber into a package system can meet the above-described need. I.e., the dual-chamber package system can provide easy access to the absorbent articles, while no additional assembling/mounting needed.

In one aspect, the present invention relates to a dual-chamber package system for absorbent articles, comprising:
a retaining chamber for vertically retaining a plurality of absorbent articles,
a dispensing chamber positioned underneath the retaining chamber, wherein the dispensing chamber has a front side opening, and
a divider dividing the retaining chamber and the dispensing chamber, wherein at least a portion of the divider is moveable so that a bottommost absorbent article, of the plurality of absorbent articles vertically retained in the retaining chamber, is dispensable from the front side opening of the dispensing chamber.

The divider is comprised of a fixed portion, a movable portion and a hinge line positioned therebetween, wherein the movable portion is the at least portion of the divider that is moveable.

The fixed portion of the divider has a 1ength L1 which is along a direction on the movable portion plane orthogonal to the hinge line.

The movable portion of the divider has a 1ength L2 which is along a direction on the movable portion plane orthogonal to the hinge line.

Further the ratio of the length L2 of the movable portion to the length L1 of the fixed portion is from 1:1 to 1:4.

In another aspect, the present invention relates to an absorbent article package, comprising one or more the dual-chamber package system set forth hereabove, and absorbent articles retained in the retaining chamber of the dual-chamber package system.

An advantage of the dual-chamber package system is that the dual-chamber package system is free to stand by itself on any surface and no need to attached to a wall. Another advantage of the dual-chamber package system is that it can serve as either a primary packaging or preferably a retail packaging for storing absorbent articles for a shelf life.

Another advantage of the dual-chamber package system is capable to provide other functionalities, such as visual attraction for moms and/or babies by incorporating artworks on the package.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings and which like designations are used to designate substantially identical elements, and in which:
Figure 1 is a schematic perspective view of one embodiment of a dual-chamber package system of the present invention in a closed configuration.
Figure 2 is a cross-sectional view along A-A' of the dual-chamber package system of Figure 1 in the closed configuration.
Figure 3 is a schematic perspective view of the embodiment of the dual-chamber package system of the present invention in an opened configuration.
Figure 4 is a cross-sectional view along A-A' of the dual-chamber package system of Figure 3 in the opened configuration.
Figure 5 is a schematic drawing illustrating the withdrawing procedure by a user.

### DETAILED DESCRIPTION OF THE INVENTION

Features and benefits of the various embodiments of the present invention will become apparent from the following description, which includes examples of specific embodiments intended to give a broad representation of the invention. Various modifications will be apparent to those skilled in the art from this description and from practice of the invention. The scope of the present invention is not intended to be limited to the particular forms disclosed and the invention covers all modifications, equivalents, and alternatives falling within the scope of the invention as defined by the claims.

As used herein, the articles including "the", "a" and "an" when used in a claim or in the specification, are understood to mean one or more of what is claimed or described.

As used herein, the terms "comprise", "comprising", "include", "including", "contain", and "containing" are meant to be non-limiting, i.e., other steps and other ingredients which do not affect the end of result can be added. The above terms encompass the terms "consisting of'.

"Absorbent article" refers to articles of wear which may be in the form of pant-type diapers, taped diapers, swim diapers, feminine hygiene articles such as feminine pads and tampons, incontinent briefs, and the like which are so configured to also absorb and contain various exudates such as urine, feces, and menses discharged from the body.

"Longitudinal" refers to a direction running substantially perpendicular from a waist edge to an opposing waist edge of the article and generally parallel to the maximum linear dimension of the article.

"Transverse" refers to a direction perpendicular to the longitudinal direction.

"Proximal" and "distal" refer respectively to the position closer or farther relative to the longitudinal center of the article.

"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable absorbent article).

"Disposed" refers to an element being located in a particular place or position.

"Joined" refers to configurations whereby an element is directly secured to another element by affixing the element directly to the other element and to configurations whereby an element is indirectly secured to another element by affixing the element to intermediate member(s) which in turn are affixed to the other element.

"Film" refers to a sheet-like material wherein the length and width of the material far exceed the thickness of the material. Typically, films have a thickness of about 0.5 mm or less.

"Water-permeable" and "water-impermeable" refer to the penetrability of materials in the context of the intended usage of disposable absorbent articles. Specifically, the term "water-permeable" refers to a layer or a layered structure having pores, openings, and/or interconnected void spaces that permit liquid water, urine, or synthetic urine to pass through its thickness in the absence of a forcing pressure. Conversely, the term "water-impermeable" refers to a layer or a layered structure through the thickness of which liquid water, urine, or synthetic urine cannot pass in the absence of a forcing pressure (aside from natural forces such as gravity). A layer or a layered structure that is water-impermeable according to this definition may be permeable to water vapor, i.e., may be "vapor-permeable".

"Extendibility" and "extensible" mean that the width or length of the component in a relaxed state can be extended or increased.

"Elasticated" and "elasticized" mean that a component comprises at least a portion made of elastic material.

"Elongatable material", "extensible material", or "stretchable material" are used interchangeably and refer to a material that, upon application of a biasing force, can stretch to an elongated length of at least about 110% of its relaxed, original length (i.e. can stretch to 10 percent more than its original length), without rupture or breakage, and upon release of the applied force, shows little recovery, less than about 20% of its elongation without complete rupture or breakage as measured by EDANA method 20.2-89. In the event such an elongatable material recovers at least 40% of its elongation upon release of the applied force, the elongatable material will be considered to be "elastic" or "elastomeric." For example, an elastic material that has an initial length of 100 mm can extend at least to 150 mm, and upon removal of the force retracts to a length of at least 130 mm (i.e., exhibiting a 40% recovery). In the event the material recovers less than 40% of its elongation upon release of the applied force, the elongatable material will be considered to be "substantially non-elastic" or "substantially non-elastomeric". For example, an elongatable material that has an initial length of 100 mm can extend at least to 150 mm, and upon removal of the force retracts to a length of at least 145 mm (i.e., exhibiting a 10% recovery).

"Artwork" refers to a visual presentation to the naked eye, which is provided by printing or otherwise, and having a color. Printing includes various methods and apparatus well known to those skilled in the art such as lithographic, screen printing, flexographic, and gravure ink jet printing techniques.

"Color" or "Colored" as referred to herein includes any primary color except color white, i.e., black, red, blue, violet, orange, yellow, green, and indigo as well as any declination thereof or mixture thereof. The color white is defined as those colors having an L* value of at least 94, an a* value equal to 0 ± 2, and a b* value equal to 0 ± 2 according to the CIE L* a* b* color system.

"Primary packaging" refers to packaging that is in direct contact with the absorbent article herein and distinguished from "retail packaging". "Retail packaging" refers to packaging that encases or encompasses the primary packaging. Retail packaging may encase one or more of the primary packaging. Any packaging that contains the primary packaging is referred to as "retail packaging", namely, a tertiary packaging that encase a secondary packaging are also collectively referred to as "retail packaging".

Relative positional terms (e.g., "bottom," "top," "side," "upper," "lower," "horizontal," "vertical," etc.) are used herein with respect to the orientation of the packaging as intended for display in retail sales or in-use environments according to artwork provided to the packaging or otherwise.

"Self-standing" refers to a state in which the packaging can maintain the intended display configuration for the length of the intended life cycle of the merchandize, typically 3 years.

As used herein, all concentrations and ratios are on a weight basis unless otherwise specified. All temperatures herein are in degrees Celsius (°C) unless otherwise indicated. All conditions herein are at 20°C and under the atmospheric pressure, unless otherwise specifically stated. All polymer molecular weights are by average number molecular weight unless otherwise specifically noted.

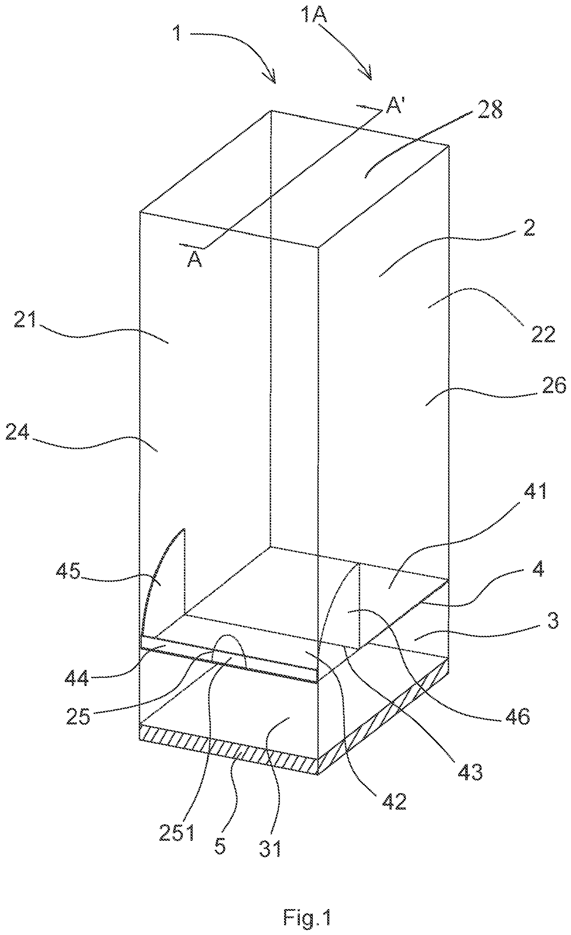

Figure 1 shows a schematic perspective view of one embodiment of a dual-chamber package system (1) of the present invention in a closed configuration (1A). The dual-chamber package system (1) comprising a retaining chamber (2) and a dispensing chamber (3). The retaining chamber (2) has a front wall (21), a back wall (22), a left wall (24), a right wall (26), and a top wall (28). The retaining chamber (2) retains a plurality of absorbent articles which are vertically packed. The dispensing chamber (3) is positioned underneath the retaining chamber (2), where the dispensing chamber (3) has a front side opening (31). The dual-chamber package system further comprises a divider (4) which divides the retaining chamber (2) and the dispensing chamber (3).

The dual-chamber package system (1) may be made by non-metal materials. The dual-chamber package system (1) may be made by reinforced pulp material typically referred to as carton or cardboard, hardened plastic such as high density polyethylene or polyethylenephthalate, or combinations thereof, which provide enough rigidity to self-stand.

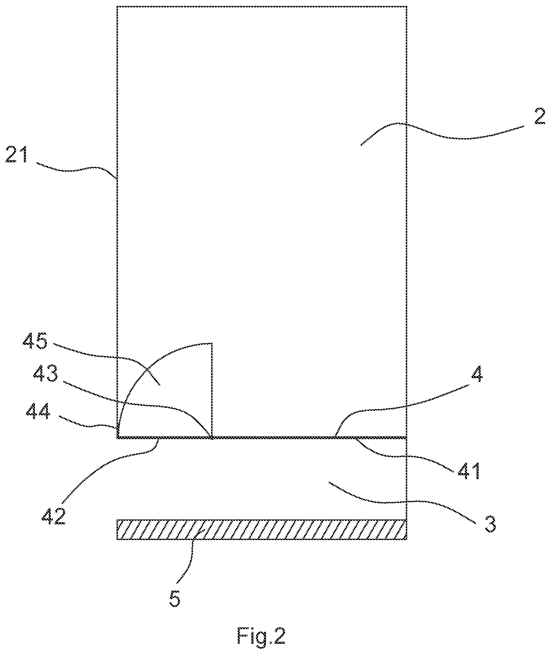

Figure 2 shows a cross-sectional view along A-A' direction of the dual-chamber package system (1) of Figure 1 in the closed configuration (1A).

Referring to both Figure 1 and Figure 2, the divider (4) comprises a fixed portion (41), a movable portion (42) and a hinge line (43) positioned therebetween, where the movable portion (42) is the at least portion of the divider that is operationally moveable. The fixed portion (41) has a fixed portion plane, and this fixed portion plane may be a horizontal plane. The movable portion (42) has a movable portion plane. The movable portion plane of the movable portion (42) is on the same plane as the fixed portion plane of the fixed portion (41) in a closed configuration.

Still referring to Figure 1 and figure 2, the movable portion (42) of the divider (4) may further comprise a front flap (44), and the front flap (44) may oppose the hinge line (43). Such front flap (44) extends along a plane (not shown) essentially orthogonal to the movable portion plane of the movable portion (42). The front flap (44) is engageable to the retaining chamber (2). In one example, the front flap (44) may be frictionally engaged with the front wall (21) of the retaining chamber (2) by inserting into the retaining chamber (2) when the movable portion (42) is in the closed position. Alternatively, the front flap (44) may also be engaged with the front wall (21) by any other suitable means. The front flap (44) of the divider (4) may be at least partially bonded with the front wall (21) of the retaining chamber (2) by e.g. glue, or pressure, or double side adhesive tape etc.

The movable portion (42) of the divider (4) may further comprise at least a first side flap (45) on one side of the movable portion (42) that extends along a plane (not shown) essentially orthogonal to the movable portion plane of the movable portion (42). The movable portion (42) of the divider (4) may further comprise a second side flap (46) on another side of the movable portion (42) opposing to the first side flap (45) that extends along a plane essentially orthogonal to the movable portion plane of the movable portion (42). Herein the term "essentially" is a recognition that the flap may not be completely vertically, i.e., 90 degrees relative to the movable portion plane but rather there could be some tolerances or design modifications so that the "flap" is +/- 15 degrees from being vertical. Referring to Figure 1, when the movable portion (42) is in the closed position, the first side flap (45) is adjacent to the left wall (24) of the retaining chamber (2), while the second side flap (46) is adjacent to the right wall (26) of the retaining chamber (2). The first side flap (45) may be frictionally engaged with the left wall (24) of the retaining chamber (2), and the second side flap (46) is frictionally engaged with the right wall (26) of the retaining chamber (2). Herein the left wall (24) and the right wall (26) is referred by viewing from facing the front opening of the dispensing chamber. The intersection of the first side flap (45) and the movable portion (42) may extend through either a part of or the whole of side length of the movable portion (42). The shape of the first or second side flap may be of any suitable shape, e.g. a half-circle, quarter-circle, etc.

The front wall (21) of the retaining chamber (2) may comprise a line of weakness (25) at the bottom portion of the front wall (21). The line of weakness (25) may be positioned in the central part of the bottom portion of the front wall (21). The line of weakness (25) may be configured to form a thumb-shape opening (252) at the lower edge of the front wall (21). The thumb-shaped opening (252) may have a height equal to or exceeding the thickness of one piece of the absorbent article but smaller than twice the thickness thereof.

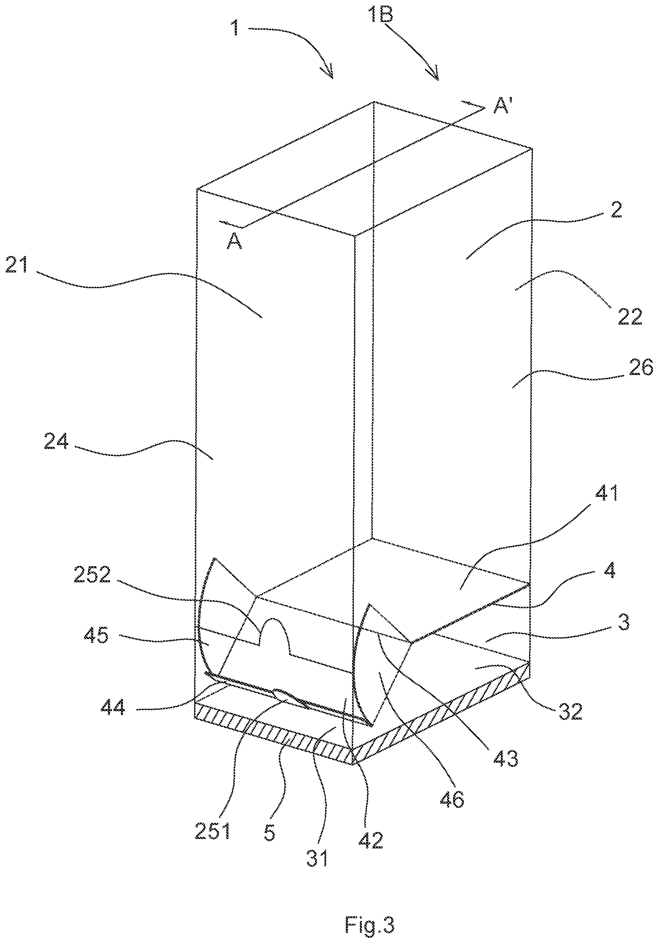

Figure 3 shows a schematic perspective view of the embodiment of the dual-chamber package system (1) of the present invention in an opened configuration (1B).

The movable portion plane of the movable portion (42) may move downward along the hinge line (43) to reach an opened position from the closed position, at which the fixed portion plane and the movable portion plane forms an angle (θ) of less than 180 degree, or from about 90 degree to about 165 degree, or from about 120 degree to about 150 degree.

Upon the opening action, the front flap (44) may be disengaged from the front wall (21) of the retaining chamber (2) and move downward together with the movable portion (42) while maintaining essentially orthogonal to the movable portion plane of the movable portion (42). The first and second side flap (45, 46) may move downward along the hinge line (43) together with the movable portion (42) while maintaining essentially orthogonal to the movable portion plane of the movable portion (42). The side flap(s) may help protect the absorbent articles from being exposed to outside environment to avoid any potential contamination.

One approach for opening the dual-chamber package system (1) is to press and open the line of weakness (25) on the front wall (21). Upon pressing and open the line of weakness (25), a tab (251) is formed which is attached to the movable portion (42) by structurally engaging with the front flap (44) of the divider (4) after being disconnected from the front wall (21).

The dual-chamber package system (1) may further comprise a footer (5) forming a bottom inside surface (32) of the dispensing chamber (3), which opposes the bottom surface of the fixed portion (41) of the divider (4)). The footer (5) may have a height of about 0.5 cm to about 2 cm, or from about 0.8 cm to about 1.5 cm. The height is measured between the bottom inside surface (32) of the dispensing chamber (3) and the outer bottom surface of the dual-chamber package system. The footer (5) is provided to prevent the bottommost absorbent article (100) pulled out of the retaining chamber (2) through the dispensing chamber (3) from touching the surface on which the dual-chamber package system (1) stands.

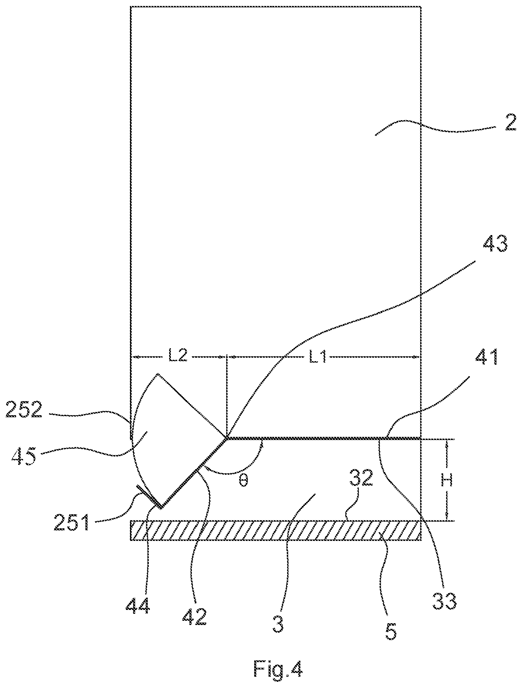

Figure 4 shows a cross-sectional view along A-A' of the dual-chamber package system (1) of Figure 3 in the opened configuration (1B).

The fixed portion (41) of the divider (4) has a length (L1) which is along a direction on the movable portion plane orthogonal to the hinge line (43), and preferably is the distance between the hinge line (43) and the back wall (22) of the retaining chamber (2). The movable portion (42) of the divider (4) has a length (L2) which is along a direction on the movable portion plane orthogonal to the hinge line (43), preferably is the distance between the hinge line (43) and the front wall of the retaining chamber (2). The ratio of the length (L2) of the movable portion (42) to the length (L1) of the fixed portion (41) is from about 1:1 to about 1:4. For example, ratio of L2:L1 could be about 1:3. If the length L2 is too short, the user has difficulty to put fingers to seize the bottommost absorbent article. If the length L2 is too long, the bottommost absorbent article may fall out of the retaining chamber (2) into the dispensing chamber (3).

The front side opening (31) of the dispensing chamber (3) has a height (H) which is the distance between the upper inside surface (32) to the bottom inside surface (33) of the dispensing chamber (3), i.e. from the bottom surface of the fixed portion (41) of the divider (4) to the top surface of the footer (5). In such case, upon opening, the movable portion (42) of the divider (4) abuts the bottom inside surface (33) of the dispensing chamber (3) where the angle (θ) between the movable portion plane of the movable portion (42) with the fixed portion plane of the fixed portion (41) may be from more than about 90 degree to about 150 degree.

In some embodiments, the absorbent articles are contained in a primary packaging which is further retained in the retaining chamber (2) of the dual-chamber package system (1). That is, the retaining chamber (2) contains a primary packaging housing a plurality of absorbent articles. Herein "primary packaging" refers to packaging that is in direct contact with the absorbent article. For articles such as feminine pads and tampons which may be housed in, or covered by an individual applicator/release paper/film, such applicator/release paper/film is considered part of the absorbent article.

The primary packaging may be formed of a single layer or multilayer polyolefin film having a desirable combination of relatively low material cost, strength and weldability at relatively low temperatures. When a multilayer film is used, the outer and inner layers may be co-formed (such as by co-extrusion), or in another example, may be separately formed and then laminated together following their formation, by use of a suitable laminating adhesive. In this latter example, an advantage provided is that one of the layers may be printed on one side before lamination. Following that, the printed side may be faced inward (facing the other layer(s)) during lamination, such that it is protected by the other layer(s) from abrasion and wear in the finished film product, thereby preserving the integrity of the printed images, graphics, verbal content, etc. A suitable multilayer film may be formed of one or more polyolefins, such as polypropylene and polyethylene. In one example, the stock film may have at least two layers, including a first layer of polyethylene and second layer of polypropylene. In one example, a layer formed of polypropylene having a first relatively higher melting temperature, and a layer of polyethylene having a second relatively lower melting temperature, may be used to form the outer and inner layers, respectively. The absorbent articles could be diapers, or feminine hygiene articles. When the absorbent article is a diaper, a pre-determined number of diapers are folded, stacked, and compressed, prior to being enclosed in primary packaging formed of polymer film. For example, the diapers are each folded at a transverse centerline and stacked in the retaining chamber (2) with the fold line towards the front wall. For purposes of efficiency in shipment, storage, and display, a certain amount of compression is applied to the stack of diapers. The amount of compression to be applied may be determined according to the number of diaper pads to be included per package, and in consideration of the influence of the compression force applied to the diaper. The in-bag compression of the package of the present invention is typically in the range of from about 1000 Pa to about 10000 Pa. The primary packaging may accommodate from about 5 to about 50 articles. For sake of manufacturing efficiency, the primary packaging may be the same as those regularly supplied to the market for daily usage.

The primary packaging may be provided with a line of weakness, by perforation or otherwise, for enabling the user to tear open the film for retrieving the absorbent articles from the primary packaging. In a situation where the retaining chamber (2) contains a primary packaging which accommodates a plurality of absorbent articles, the line of weakness provided on the primary packaging is at least partially overlapping with and preferably further structurally attached to the line of weakness (25) at the lower edge of the front wall (21) of the retaining chamber (2), so that these overlapped and structurally attached two layers of line of weaknesses can be torn open together by a user.

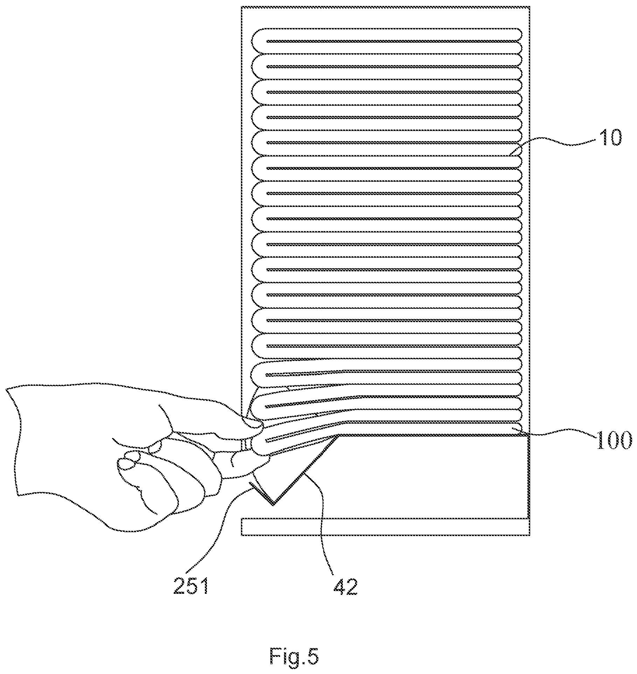

Figure 5 is a schematic drawing illustrating the withdrawing procedure by a user. Folded diapers are drawn as exemplary absorbent articles (10) and the number of diapers in the figure is only for purpose of illustration. By pressing the line of weakness, a tab (251) configured by the line of weakness together with the movable portion (42) of the divider is disconnected from the front edge of the retaining chamber, and move downwards. The thumb is introduced through the opening configured by the line of weakness and separates the bottommost diaper (100) from the second bottommost diaper. The bottommost diaper (100) is seized between thumb and the fingers, slightly tilted downwards in the seized region, and then withdrawn at an angle in forward/downward directions through the dispensing chamber. The second bottommost diaper falls due to gravity.

The dual-chamber package system (1) may further comprise an artwork, the information selected from the group consisting of: visual presentation, information for using the absorbent article, developmental information associated with the absorbent article, developmental information unassociated with the absorbent article, and combinations thereof. The information provided in each artwork is selected according to the purpose of the package system and the types of absorbent articles to be housed in each package system. The unique artwork may be one associated with the brand of the merchandize, a character, or any other attractive visual presentation. For example, the unique artwork could be a drawing or figure of an animal, such as panda, elephant, cat, dolphin, or Chinese Zodiacs.

The artwork may be provided at least on the front wall (21) of the retaining chamber (2). In a preferred embodiment, the line of weakness (25) on the front wall (21) of the retaining chamber (2) constitutes part of an artwork on the front wall. For example, the line of weakness (25) may constitute a mouth of a panda artwork, upon tearing of the tab (251) off the front wall, the thumb-shape opening is showed as an open mouth of a smiling panda.

The dual-chamber package system (1) could be cuboids or block shapes, wherein the retaining chamber (2) is preferably a cuboid or block shape. In some embodiments, the dual-chamber package system (1) has a horizontal cross section of rectangular shape, having a width of from about 5 cm to about 40 cm, or from about 15 cm to about 30 cm, or from about 18 cm to about 26 cm; and a length of from about 5 cm to about 40 cm, or from about 15 cm to about 35 cm, or from about 25 cm to about 33 cm. Such dimensions are suitable for receiving e.g. once-folded diapers and ensuring the absorbent articles stacking reliably. The total height of the dual-chamber package system (1) is from about 20 cm to about 60 cm, or from about 35 cm to about 55 cm, or from about 38 cm to about 52 cm. The retaining chamber (2) may have a height from about 20 cm to about 50 cm, or from about 35 cm to about 50 cm, to receiving a certain number of vertically stacked diapers, e.g. about 5 to about 50 diapers.

The dual-chamber package system (1) may further comprise a handle, preferably positioned at either the top wall (28) or the back wall (22) of the retaining chamber (2). Preferably, the handle may be made of any suitable materials and may be made to any forms so long as it may be handled. For example, the handle may be made by the same material as the dual-chamber package system. Alternatively, the handle may be made by ribbons, elastic material, and otherwise. Handle having ornamental features such as ribbons and elastic material may be attractive for certain purposes.

The dual-chamber package system (1) may further accommodate in the dispensing chamber (3) articles other than absorbent articles and associated with child bearing, such as wipes, clothing, bibs, toys, and otherwise.

## Claims

1. A dual-chamber package system (1) for absorbent articles, comprising:
a retaining chamber (2) for vertically retaining a plurality of absorbent articles (10),
a dispensing chamber (3) positioned underneath the retaining chamber (2), wherein the dispensing chamber (3) has a front side opening (31), and
a divider (4) dividing the retaining chamber (2) and the dispensing chamber (3),
wherein at least a portion of the divider (4) is moveable so that a bottommost absorbent article (100), of the plurality of absorbent articles (10) vertically retained in the retaining chamber (2), is dispensable from the front side opening (31) of the dispensing chamber (3),
**characterized in that**
the divider (4) is comprised of a fixed portion (41), a movable portion (42) and a hinge line (43) positioned therebetween,
wherein the movable portion (42) is the at least portion of the divider that is moveable;
wherein the fixed portion (41) of the divider (4) has a 1ength (L1) which is along a direction on the movable portion plane orthogonal to the hinge line (43);
wherein the movable portion (42) of the divider (4) has a 1ength (L2) which is along a direction on the movable portion plane orthogonal to the hinge line (43); and
wherein the ratio of the length (L2) of the movable portion (42) to the length (L1) of the fixed portion (41) is from 1: 1 to 1:4.

2. The dual-chamber package system (1) for absorbent articles according to claim 1,
wherein the fixed portion (41) has a fixed portion plane,
wherein the movable portion (42) has a movable portion plane,
wherein the movable portion plane of the movable portion (42) is on the same plane as the fixed portion plane of the fixed portion (41) in a closed position, and moves downward along the hinge line (43) to reach an opened position, at which the fixed portion plane and the movable portion plane forms an angle less than 180 degree, preferably from 90 degree to 165 degree, more preferably from 120 degree to 150 degree.

3. The dual-chamber package system (1) for absorbent articles according to claims 1 or 2,
wherein the movable portion (42) of the divider (4) further comprises a front flap (44) that extends along a plane essentially orthogonal to the movable portion plane of the movable portion (42),
wherein the front flap (44) is engageable to the retaining chamber (2).

4. The dual-chamber package system (1) for absorbent articles according to any of the preceding claims,
wherein the movable portion (42) of the divider (4) further comprises at least a first side flap (45) on one side of the movable portion (42) that extends along a plane essentially orthogonal to the movable portion plane of the movable portion (42);
preferably wherein the movable portion (42) of the divider (4) further comprises a second side flap (46) on another side of the movable portion (42) opposing to the first side flap (45) that extends along a plane essentially orthogonal to the movable portion plane of the movable portion (42).

5. The dual-chamber package system (1) for absorbent articles according to any of the preceding claims,
wherein the dispensing chamber (3) further comprises a footer (5) forming the bottom inside surface (32) of the dispensing chamber (3),
wherein the footer has a height of 0.5 cm to 2 cm, preferably from 0.8 cm to 1.5 cm.

6. The dual-chamber package system (1) for absorbent articles according to any of the preceding claims,
wherein the retaining chamber (2) comprises a front wall (21), at the bottom of which provides a line of weakness (25), said line of weakness (25) is configured to form a tab (251) which is attached to the movable portion (42) by structurally engaging with the front flap (44) of the divider (4) after be disconnected from the front wall (21); preferably the line of weakness (25) is positioned in the central part of the bottom of the front wall (21).

7. The dual-chamber package system (1) for absorbent articles according to any of the preceding claims, wherein the length (L2) of the movable portion (42) is smaller or equal to the height (H) of the front side opening (31) of the dispensing chamber (3).

8. The dual-chamber package system (1) for absorbent articles according to any of the preceding claims, wherein the dual-chamber package system (1) has a horizontal cross section of rectangular shape, having a width of 5 cm to 40 cm, preferably from 15 cm to 30 cm, and more preferably from 18 cm to 26 cm; and a length of 5 cm to 40 cm, preferably from 15 cm to 35 cm, more preferably from 25 cm to 33 cm.

9. The dual-chamber package system (1) for absorbent articles according to any of the preceding claims, wherein the retaining chamber (2) further comprises an artwork at least on the front wall (21), preferably the line of weakness (25) constitutes part of the artwork.

10. The dual-chamber package system (1) for absorbent articles according to any of the preceding claims, further comprising a handle, preferably positioned at either the top wall (28) or the back wall (22) of the retaining chamber (2).

11. The dual-chamber package system (1) for absorbent articles according to any of the preceding claims, further comprising a primary packaging which accommodates the absorbent articles, wherein preferably the absorbent articles are diapers.

12. The dual-chamber package system (1) for absorbent articles according to any of the preceding claims, where the dual-chamber package system (1) is made of non-metal material, preferably is made of materials selected from the group consisting of carton or cardboard, hardened plastic such as high density polyethylene or polyethylenephthalate, or combinations thereof.

13. An absorbent article package, comprising one or more the dual-chamber package system (1) according to any of the preceding claims, and absorbent articles retained in the retaining chamber (2).

## Patentansprüche

1. Zweikammerverpackungssystem (1) für Absorptionsartikel, umfassend:
eine Haltekammer (2) zum vertikalen Halten einer Vielzahl von Absorptionsartikeln (10),
eine Abgabekammer (3), die unterhalb der Haltekammer (2) angeordnet ist, wobei die Abgabekammer (3) eine vorderseitige Öffnung (31) aufweist, und
einen Teiler (4), der die Haltekammer (2) und die Abgabekammer (3) teilt,
wobei mindestens ein Abschnitt des Teilers (4) bewegbar ist, derart, dass ein unterster Absorptionsartikel (100) der Vielzahl von Absorptionsartikeln (10), die vertikal in der Haltekammer (2) gehalten werden, von der vorderseitigen Öffnung (31) der Abgabekammer (3) abgebbar ist,
**dadurch gekennzeichnet, dass**
der Teiler (4) aus einem festen Abschnitt (41), einem bewegbaren Abschnitt (42) und einer dazwischen angeordneten Scharnierlinie (43) besteht,
wobei der bewegbare Abschnitt (42) mindestens der Abschnitt des Teilers ist, der bewegbar ist;
wobei der feste Abschnitt (41) des Teilers (4) eine Länge (L1) aufweist, die entlang einer Richtung auf der bewegbaren Abschnittsebene orthogonal zu der Scharnierlinie (43) ist;
wobei der bewegbare Abschnitt (42) des Teilers (4) eine Länge (L2) aufweist, die entlang einer Richtung an dem bewegbaren Abschnitt der Ebene zu der Scharnierlinie (43) orthogonal verläuft; und
wobei das Verhältnis der Länge (L2) des bewegbaren Abschnitts (42) zu der Länge (L1) des festen Abschnitts (41) von 1:1 bis 1:4 ist.

2. Zweikammerverpackungssystem (1) für Absorptionsartikel nach Anspruch 1,
wobei der feste Abschnitt (41) eine feste Abschnittsebene aufweist,
wobei der bewegbare Abschnitt (42) eine bewegbare Abschnittsebene aufweist,
wobei die bewegbare Abschnittsebene des bewegbaren Abschnitts (42) auf derselben Ebene wie die feste Abschnittsebene des festen Abschnitts (41) in einer geschlossenen Position ist und sich entlang der Scharnierlinie (43) abwärts bewegt, um eine geöffnete Position zu erreichen, an der die feste Abschnittsebene und die bewegbare Abschnittsebene einen Winkel kleiner als 180 Grad, vorzugsweise von 90 Grad bis 165 Grad, mehr bevorzugt von 120 Grad bis 150 Grad, bilden.

3. Zweikammerverpackungssystem (1) für Absorptionsartikel nach den Ansprüchen 1 oder 2,
wobei der bewegbare Abschnitt (42) des Teilers (4) ferner eine vorderseitige Klappe (44) umfasst, die sich entlang einer Ebene zu der bewegbaren Abschnittsebene des bewegbaren Abschnitts (42) im Wesentlichen orthogonal erstreckt,
wobei die vorderseitige Klappe (44) zu der Haltekammer (2) in Eingriff bringbar ist.

4. Zweikammerverpackungssystem (1) für Absorptionsartikel nach einem der vorstehenden Ansprüche,
wobei der bewegbare Abschnitt (42) des Teilers (4) ferner mindestens eine erste Seitenklappe (45) an einer Seite des bewegbaren Abschnitts (42) umfasst, die sich entlang einer Ebene zu der bewegbaren Abschnittsebene des bewegbaren Abschnitts (42) im Wesentlichen orthogonal erstreckt;
vorzugsweise, wobei der bewegbare Abschnitt (42) des Teilers (4) ferner eine zweite Seitenklappe (46) an einer anderen Seite des bewegbaren Abschnitts (42), gegenüberliegend der ersten Seitenklappe (45), umfasst, die sich entlang einer Ebene erstreckt, die zu der bewegbaren Abschnittsebene des bewegbaren Abschnitts (42) im Wesentlichen orthogonal verläuft.

5. Zweikammerverpackungssystem (1) für Absorptionsartikel nach einem der vorstehenden Ansprüche,
wobei die Abgabekammer (3) ferner einen Fuß (5) umfasst, der die untere Innenoberfläche (32) der Abgabekammer (3) ausbildet,
wobei der Fuß eine Höhe von 0,5 cm bis 2 cm, vorzugsweise von 0,8 cm bis 1,5 cm aufweist.

6. Zweikammerverpackungssystem (1) für Absorptionsartikel nach einem der vorstehenden Ansprüche,
wobei die Haltekammer (2) eine Vorderwand (21), an deren Unterseite eine Schwächungslinie (25) bereitgestellt ist, umfasst, wobei die Schwächungslinie (25) konfiguriert ist, um eine Lasche (251) auszubilden, die durch ein konstruktives Ineingriffnehmen mit der vorderen Klappe (44) des Teilers (4) an dem bewegbaren Abschnitt (42) angebracht ist, nachdem sie von der Vorderwand (21) getrennt ist; wobei vorzugsweise die Schwächungslinie (25) in dem zentralen Teil des Bodens der Vorderwand (21) angeordnet ist.

7. Zweikammerverpackungssystem (1) für Absorptionsartikel nach einem der vorstehenden Ansprüche, wobei die Länge (L2) des bewegbaren Abschnitts (42) kleiner oder gleich zu der Höhe (H) der vorderseitigen Öffnung (31) der Abgabekammer (3) ist.

8. Zweikammerverpackungssystem (1) für Absorptionsartikel nach einem der vorstehenden Ansprüche, wobei das Zweikammerverpackungssystem (1) einen horizontalen Querschnitt mit rechteckiger Form aufweist, der eine Breite von 5 cm bis 40 cm, vorzugsweise von 15 cm bis 30 cm und mehr bevorzugt von 18 cm bis 26 cm; und eine Länge von 5 cm bis 40 cm, vorzugsweise von 15 cm bis 35 cm, mehr bevorzugt von 25 cm bis 33 cm aufweist.

9. Zweikammerverpackungssystem (1) für Absorptionsartikel nach einem der vorstehenden Ansprüche, wobei die Haltekammer (2) ferner eine Illustration mindestens an der Vorderwand (21) umfasst, wobei vorzugsweise die Schwächungslinie (25) einen Teil des Illustration ausmacht.

10. Zweikammerverpackungssystem (1) für Absorptionsartikel nach einem der vorstehenden Ansprüche, ferner umfassend einen Griff, der vorzugsweise entweder an der oberen Wand (28) oder der Hinterwand (22) der Haltekammer (2) angeordnet ist.

11. Zweikammerverpackungssystem (1) für Absorptionsartikel nach einem der vorstehenden Ansprüche, ferner umfassend eine Primärverpackung, die die Absorptionsartikel aufnimmt, wobei vorzugsweise die Absorptionsartikel Windeln sind.

12. Zweikammerverpackungssystem (1) für Absorptionsartikel nach einem der vorstehenden Ansprüche, wobei das Zweikammerverpackungssystem (1) aus Nichtmetallmaterial hergestellt ist, wobei es vorzugsweise aus Materialien hergestellt ist, die aus der Gruppe bestehend aus Karton oder Pappe, gehärtetem Kunststoff, wie Polyethylen hoher Dichte oder Polyethylenphthalat oder Kombinationen davon ausgewählt sind.

13. Absorptionsartikelverpackung, umfassend ein oder mehrere des Zweikammerverpackungssystems (1) nach einem der vorstehenden Ansprüche und Absorptionsartikel, die in der Haltekammer (2) zurückgehalten werden.

## Revendications

1. Système de conditionnement à double chambre (1) pour articles absorbants, comprenant :
une chambre de retenue (2) destinée à retenir verticalement une pluralité d'articles absorbants (10),
une chambre de distribution (3) positionnée en dessous de la chambre de retenue (2), dans lequel la chambre de distribution (3) a une ouverture côté avant (31), et
un séparateur (4) séparant la chambre de retenue (2) et la chambre de distribution (3),
dans lequel au moins une partie du séparateur (4) est déplaçable de sorte qu'un article absorbant le plus au fond (100), de la pluralité d'articles absorbants (10) retenus verticalement dans la chambre de retenue (2), peut être distribué à partir de l'ouverture côté avant (31) de la chambre de distribution (3),
**caractérisé en ce que**
le séparateur (4) se compose d'une partie fixe (41), d'une partie mobile (42) et d'une ligne charnière (43) positionnée entre elles,
dans lequel la partie mobile (42) est l'au moins une partie du séparateur qui est déplaçable ;
dans lequel la partie fixe (41) du séparateur (4) a une longueur (L1) qui est le long d'une direction sur le plan de partie mobile orthogonale à la ligne charnière (43) ;
dans lequel la partie mobile (42) du séparateur (4) a une longueur (L2) qui est le long d'une direction sur le plan de partie mobile orthogonale à la ligne charnière (43) ; et
dans lequel le rapport de la longueur (L2) de la partie mobile (42) à la longueur (L1) de la partie fixe (41) est de 1:1 à 1:4.

2. Système de conditionnement à double chambre (1) pour articles absorbants selon la revendication 1,
dans lequel la partie fixe (41) a un plan de partie fixe,
dans lequel la partie mobile (42) a un plan de partie mobile,
dans lequel le plan de partie mobile de la partie mobile (42) est sur le même plan que le plan de partie fixe de la partie fixe (41) dans une position fermée, et se déplace vers le bas le long de la ligne charnière (43) pour atteindre une position ouverte, au niveau de laquelle le plan de partie fixe et le plan de partie mobile forme un angle inférieur à 180 degrés, de préférence de 90 degrés à 165 degrés, plus préférablement de 120 degrés à 150 degrés.

3. Système de conditionnement à double chambre (1) pour articles absorbants selon les revendications 1 ou 2,
dans lequel la partie mobile (42) du séparateur (4) comprend en outre un rabat avant (44) qui s'étend le long d'un plan pratiquement orthogonal au plan de partie mobile de la partie mobile (42),
dans lequel le rabat avant (44) peut venir en prise avec la chambre de retenue (2).

4. Système de conditionnement à double chambre (1) pour articles absorbants selon l'une quelconque des revendications précédentes,
dans lequel la partie mobile (42) du séparateur (4) comprend en outre au moins un premier rabat latéral (45) sur un côté de la partie mobile (42) qui s'étend le long d'un plan pratiquement orthogonal au plan de partie mobile de la partie mobile (42) ;
de préférence dans lequel la partie mobile (42) du séparateur (4) comprend en outre un second rabat latéral (46) sur un autre côté de la partie mobile (42) opposé au premier rabat latéral (45) qui s'étend le long d'un plan pratiquement orthogonal au plan de partie mobile de la partie mobile (42).

5. Système de conditionnement à double chambre (1) pour articles absorbants selon l'une quelconque des revendications précédentes,
dans lequel la chambre de distribution (3) comprend en outre une semelle (5) formant la surface intérieure de fond (32) de la chambre de distribution (3),
dans lequel la semelle a une hauteur de 0,5 cm à 2 cm, de préférence de 0,8 cm à 1,5 cm.

6. Système de conditionnement à double chambre (1) pour articles absorbants selon l'une quelconque des revendications précédentes,
dans lequel la chambre de retenue (2) comprend une paroi avant (21), dont le fond fournit une ligne de faiblesse (25), ladite ligne de faiblesse (25) est conçue pour former une languette (251) qui est fixée à la partie mobile (42) par mise en prise structurellement avec le rabat avant (44) du séparateur (4) après avoir été démontée de la paroi avant (21) ; de préférence la ligne de faiblesse (25) est positionnée dans la partie centrale du fond de la paroi avant (21).

7. Système de conditionnement à double chambre (1) pour articles absorbants selon l'une quelconque des revendications précédentes, dans lequel la longueur (L2) de la partie mobile (42) est inférieure ou égale à la hauteur (H) de l'ouverture côté avant (31) de la chambre de distribution (3).

8. Système de conditionnement à double chambre (1) pour articles absorbants selon l'une quelconque des revendications précédentes, dans lequel le système de conditionnement à double chambre (1) a une section transversale horizontale de forme rectangulaire, ayant une largeur de 5 cm à 40 cm, de préférence de 15 cm à 30 cm, et plus préférablement de 18 cm à 26 cm ; et une longueur de 5 cm à 40 cm, de préférence de 15 cm à 35 cm, plus préférablement de 25 cm à 33 cm.

9. Système de conditionnement à double chambre (1) pour articles absorbants selon l'une quelconque des revendications précédentes, dans lequel la chambre de retenue (2) comprend en outre une illustration au moins sur la paroi avant (21), de préférence la ligne de faiblesse (25) constitue une partie de l'illustration.

10. Système de conditionnement à double chambre (1) pour articles absorbants selon l'une quelconque des revendications précédentes, comprenant en outre une poignée, de préférence positionnée au niveau de la paroi supérieure (28) ou de la paroi arrière (22) de la chambre de retenue (2).

11. Système de conditionnement à double chambre (1) pour articles absorbants selon l'une quelconque des revendications précédentes, comprenant en outre un conditionnement primaire qui loge les articles absorbants, dans lequel de préférence les articles absorbants sont des couches.

12. Système de conditionnement à double chambre (1) pour articles absorbants selon l'une quelconque des revendications précédentes, où le système de conditionnement à double chambre (1) est en un matériau non métallique, de préférence est en matériaux choisis dans le groupe constitué de carton ou carton blanchi, plastique durci tel que du polyéthylène haute densité ou du téréphtalate de polyéthylène, ou des combinaisons de ceux-ci.

13. Conditionnement d'articles absorbants, comprenant un ou plusieurs du système de conditionnement à double chambre (1) selon l'une quelconque des revendications précédentes, et des articles absorbants retenus dans la chambre de retenue (2).
